# EUROPÄISCHE PATENTSCHRIFT

(11) **EP 1 429 859 B1**
(45) Veröffentlichungstag und Bekanntmachung des Hinweises auf die Patenterteilung: **07.01.2009**
(21) Anmeldenummer: 02800057.8
(22) Anmeldetag: 26.07.2002
(51) Int. Cl.: B01D 61/28, B01D 61/30, B01D 63/02, B01D 65/00, A61M 1/14, A61M 1/16, B01D 65/08

(54) **FILTERVORRICHTUNG SOWIE EINE VERWENDUNG EINES GEHÄUSES DER FILTERVORRICHTUNG**
FILTERING DEVICE AND USE OF A HOUSING OF THE DEVICE
DISPOSITIF DE FILTRATION ET UNE APPLICATION D' UN BOITIER DU DISPOSITIF

(30) Priorität: 28.09.2001 DE 10147903
(43) Veröffentlichungstag der Anmeldung: 23.06.2004
(73) Patentinhaber: Fresenius Medical Care Deutschland GmbH, 61352 Bad Homburg (DE)
(72) Erfinder: LAUER, Martin, 66606 St. Wendel (DE); HAHMANN, Uwe, 72555 Metzingen (DE); WIESEN, Gerhard, 61352 Bad Homburg (DE)
(74) Vertreter: Lorenz, Eduard
(86) Internationale Anmeldenummer: PCT/EP2002/008357
(87) Internationale Veröffentlichungsnummer: WO 2003/028859

(56) Entgegenhaltungen:
- EP-A- 0 887 100
- WO-A-01/08722
- US-A- 4 080 295

## Beschreibung

Die vorliegende Erfindung betrifft eine Filtervorrichtung mit einem Gehäuse, mit wenigstens zwei durch Membranen voneinander getrennten Strömungsräumen, mit mindestens einem Anschluß für jeden Strömungsraum, sowie mit wenigstens einer Filterkappe, die im Endbereich des Gehäuses aufgesetzt ist und mit diesem mittelbar oder unmittelbar verbunden ist. Die vorliegende Erfindung betrifft ferner eine verwendung eines Gehäuses einer Filtervorrichtung mit wenigstens zwei durch Membranen voneinander getrennten Strömungsräumen sowie mit wenigstens einem Endbereich, der mit einem der Strömungsräume in Verbindung steht und der durch eine auf das Gehäuse aufsetzbare Filterkappe verschließbar ist.

Derartige Filtervorrichtungen werden umfangreich im Bereich der Hämodialyse, Hämofiltration und Hämodiafiltration eingesetzt. Sie bestehen üblicherweise aus einem Gehäuse, in dem ein Faserbündel angeordnet ist, das aus einer Vielzahl von Hohlfasern besteht, die zwei Strömungsräume voneinander trennen. Während die Durchgänge der Hohlfasern von Blut durchströmt werden, wird der von dem Gehäuse begrenzte Faseraußenraum von Dialysat umströmt. Die Enden der Fasern sind in Vergußmassen aufgenommen, die sich in den Endbereichen des Gehäuses befinden. Die Vergußmasse wird üblicherweise in Vergußkappen im Endbereich des Gehäuses eingespritzt. Nach dem ausreichenden Erhärten der Vergußmasse wird die Vergußkappe entfernt und der Vergußschnitt vorgenommen, wodurch die Faserenden geöffnet werden. Nach dem Aufschneiden der Faserenden werden die Filterkappen auf das Gehäuse aufgesetzt.

Bei vorbekannten Filtervorrichtungen ist jeder Strömungsraum im allgemeinen mit jeweils zwei Anschlüssen versehen, durch die das jeweilige Medium (Blut / Dialysat) zu- bzw. abgeführt wird. Diese Anschlüsse befinden sich in den Filterkappen des Gehäuses, so daß das Gehäuse anschlußfrei ist. Ebenso sind Filtervorrichtungen bekannt, bei denen die Anschlüsse für das das Hohlfaserbündel umströmende Dialysat am Gehäuse und die Anschlüsse für das Blut in den Filterkappen angeordnet sind. Eine Filtervorrichtung, bei der sämtliche Anschlüsse bzw. Anschlußstutzen in den Filterkappen angeordnet sind, ist aus der EP 887 100 bekannt. Bei dieser Filtervorrichtung sind sämtliche Anschlüsse zu einer Seite der - Filtervorrichtung ausgerichtet und deren Mittellinien verlaufen parallel zueinander. Dadurch wird gewährleistet, daß sich die Filtervorrichtung einfach und zuverlässig mit einer Filter- oder Dialysemaschine bzw. einer Halterung durch Aufstecken in der Weise verbinden läßt, daß die Anschlüsse oder Anschlußstutzen der Filtervorrichtung in dichtende Verbindungen mit Gegenanschlüssen oder Gegenanschlußstutzen gelangen.

Bei einer derartigen Filtervorrichtung besteht jedoch ein Nachteil darin, daß aufgrund von Toleranzen beim Aufsetzen der Filterkappen auf das Gehäuse beim Konnektieren einer derartigen Filtervorrichtung auf die entsprechenden Gegenstücke Positionierungsprobleme auftreten. Diese können dazu führen, daß keine optimal dichtende Verbindung zwischen den Anschlüssen bzw. Anschlußstutzen der Filtervorrichtung und den entsprechenden Gegenstücken vorliegt, was eine nicht zufriedenstellende Funktion der Filtervorrichtung und eine Gefährdung des Patienten zur Folge haben kann.

Um derartige Probleme bei der Positionierung zu vermeiden, wird in der EP 887 100 vorgeschlagen, mittels eines Zentrierstiftes des die Filtervorrichtung aufnehmenden Halteteils, den Abstand der Anschlüsse des Halteteils genau so einzustellen, daß dieser mit dem Abstand der Anschlüsse an der Filtervorrichtung übereinstimmt. Auf diese Weise ist eine zuverlässige Konnektierung möglich, jedoch ist eine derartige Korrektur verhältnismäßig aufwendig.

Aus der U.S. 4,211,597 ist eine Filtervorrichtung bekannt, die nicht aus einem Gehäuse und darauf aufgesetzten Filterkäppen besteht, sondern aus zwei Hälften, die in Längsrichtung miteinander verbunden werden und die das Hohlfaserbündel aufnehmen. Bei der Herstellung derartiger Filtervorrichtungen wird in gesonderten Arbeitsschritten zunächst das Filtergehäuse und separat davon das Hohlfaserbündel gefertigt. In einem weiteren Fertigungsschritt wird das Hohlfaserbündel in die Gehäusehälften eingesetzt und diese fluiddicht miteinander verbunden.

Es ist die Aufgabe der vorliegenden Erfindung, eine gattungsgemäße Filtervorrichtung dahingehend weiterzubilden, daß die genannten Positionierungsprobleme beim Aufsetzen der Filtervorrichtung auf eine Halterung nicht auftreten.

Diese Aufgabe wird ausgehend von einer gattungsgemäßen Filtervorrichtung dadurch gelöst, daß sämtliche Anschlüsse für die Strömungsräume an dem Gehäuse angeordnet sind. Der Abstand der Anschlüsse bzw. Anschlußstutzen an der Filtervorrichtung ist auf diese Weise genau bestimmt, so daß die genannten Positionierungsprobleme nicht auftreten können. Toleranzschwankungen bei der Herstellung der Filterkappen oder beim Aufsetzen der Filterkappen auf das Gehäuse spielen für eine optimale Konnektion mit den Gegenstücken einer Halterung keine Rolle.

Bei der Herstellung der erfindungsgemäßen Filtervorrichtung wird in das Gehäuse die Vergußmasse eingespritzt, wobei eine Vergußkappe aufgesetzt wird, die das bzw. die Enden des Gehäuses abdichten. Nach Einspritzung der Vergußmasse sowie deren Aushärtung wird diese Kappe entfernt und die Faserenden aufgeschnitten. Nunmehr wird/werden die Endkappe(n) aufgesetzt, wobei es erfindungsgemäß auf Toleranzschwankungen bei diesem Arbeitsschritt sowie auch bei der Herstellung der Endkappe(n) nicht entscheidend ankommt. Der Abstand der Anschlüsse sowie deren Ausrichtung ist von der Ausführung und Anordnung der Filterkappen unabhängig, da sämtliche Anschlüsse erfindungsgemäß am Gehäuse angeordnet sind.

In weiterer Ausgestaltung der vorliegenden Erfindung ist vorgesehen, daß das Gehäuse rohrabschnittförmig ausgeführt ist und daß zwei Filterkappen vorgesehen sind, die auf beide Endbereiche des Gehäuses aufgesetzt und mit diesem verbunden sind. Das Gehäuse ist in diesem Fall als Hohlzylinder ausgeführt, der in seinen beiden Endbereichen mit Filterkappen verschlossen ist. Erfindungsgemäß weisen die Filterkappen keine Anschlüsse auf, da diese sämtlich am Gehäuse angeordnet sind.

In weiterer Ausgestaltung der vorliegenden Erfindung sind je Strömungsraum zwei Anschlüsse vorgesehen. Dabei dient je ein Anschluß als Zulauf für das zu reinigende Blut bzw. das frische Dialysat und der andere Anschluß jeweils als Ablauf für das gereinigte Blut bzw. das abzuführende Dialysat.

In den Endbereichen des Gehäuses können je zwei Anschlüsse angeordnet sein, die in Längsrichtung des Gehäuses jeweils übereinander oder nebeneinander angeordnet sind. In beiden Fällen steht jeweils einer der Anschlüsse mit der blutdurchströmten Kammer und jeweils der andere der Anschlüsse mit dem Dialysatraum in Verbindung. Dies kann beispielsweise -dadurch erreicht werden, daß die Anschlüsse in einem an dem Gehäuse angeformten Vorsprung aufgenommen sind, und daß sich innerhalb des Vorsprunges entsprechende mit den Anschlüssen sowie den Strömungsräumen in Verbindung stehende Leitungen erstrecken.

In weiterer Ausgestaltung der vorliegenden Erfindung ist vorgesehen, daß die Anschlüsse Stutzen aufweisen, die sich relativ zum Gehäuse in radialer oder tangentialer Richtung erstrecken. Bei tangentialer Anordnung der Stutzen kann durch entsprechende Ausgestaltung der Vergußmasse mit Blutführungskanälen sichergestellt werden, daß sich das Blut weitgehend gleichmäßig über die Enden der Hohlfasern verteilt.

Die Blutanschlußstutzen können tangential ausgeführt sein und in einen Blutführungskanal münden, der sich zumindest teilumfangsförmig um die das Hohlfaserbündel der Filtervorrichtung einfassende Vergußmasse erstreckt. Das Blut durchströmt den Blutanschlußstutzen und anschließend den Blutführungskanal, der sich teilumfangsförmig um die Vergußmasse erstreckt und mittels dessen das Blut auf die Schnittfläche der Vergußmasse und somit zu den offenen Enden der Hohlfasern gelangt. Eine tangentiale Ausführung ist selbstverständlich auch für den Dialysatanschluß möglich. Auch hier läßt sich durch geeignete Anordnung des Anschlusses eine günstige Umströmung des Faseraußenraumes mit Dialysat und damit ein optimaler Stoffaustausch über die Membranen erzielen.

Die Endbereiche des Gehäuses können im Durchmesser vergrößert sein und einen frei auskragenden Vorsprung kleineren Durchmessers aufweisen, der in der eine ein Hohlfaserbündel aufnehmende Vergußmasse eingebettet ist. Auf diese Weise läßt sich ohne weitere Dichtungsmittel eine Abdichtung des Dialysatraumes erzielen. Die Dichtung erfolgt durch Eingießen des frei auskragenden Vorsprungs in die Vergußmasse. Der Vorsprung kann einen zwangsentformten Hinterschnittkragen aufweisen, der sich in die üblicherweise aus Polyurethan bestehende Vergußmasse erstreckt. Der Vorsprung besteht üblicherweise aus Polypropylen. Die Abdichtung zwischen Blut und Dialysatseite erfolgt durch genaues Anliegen der Vergußmasse an dem Vorsprung durch den Schrumpf des Vergusses nach dessen Herstellung. Der frei auskragende Vorsprung kann beispielsweise durch eine Plasmabehandlung besonders haftfähig gemacht sein. Außer der Zylinderform sind auch weitere Geometrien, wie z. B. ein "Faltenstern" denkbar.

Die Filterkappen können mit dem Gehäuse verklebt, verschweißt oder verschraubt sein.

In bevorzugter Ausgestaltung der vorliegenden Erfindung sind im oder an dem Gehäuse Funktionselemente integriert, mittels derer Eigenschaften der durch die Filtervorrichtung geführten Medien erfaßbar und/oder veränderbar sind oder der Fluß durch die Filtervorrichtung beeinflußbar ist.

Derartige Funktionselemente können Kanäle, Ventile, Pumpen, Meßkammern, Filter und Entlüftungskammern und -membranen umfassen. Auf diese Weise läßt sich ein hochintegriertes Disposable bereitstellen, das nicht nur die Funktion der Filtration, sondern darüber hinaus weitere wichtige Funktionen, wie z. B. die Entlüftung, die Filtration oder auch das Pumpen übernimmt. Derartige Funktionselemente waren bisher im allgemeinen außerhalb des Dialyse-Disposables angeordnet, wobei die entsprechend zahlreichen Verbindungen zwischen den einzelnen Bauelementen nicht nur eine aufwendige Herstellung und Zusammenbau bedingen, sondern entsprechend auch Dichtungsprobleme mit sich bringen. Die Funktionselemente können im Gehäuse selbst oder auch an dem Gehäuse angeordnet sein. Auf diese Weise entsteht ein viele Funktionen beinhaltendes Spritzgußteil. Ein derartiges Disposable besteht aus einem einzigen Grundkörper, ist daher sehr materialsparend und insbesondere wegen der entfallenden Verbindungsprozesse fertigungs- und bedienungsfreundlich und darüber hinaus gut abdichtend.

Das Gehäuse sowie die Filterkappen können aus Polypropylen gefertigt sein.

In weiterer Ausgestaltung der vorliegenden Erfindung ist vorgesehen, daß die Anschlüsse Stutzen aufweisen, deren Endbereiche in zueinander parallelen Ebenen oder in einer gemeinsamen Ebene liegen. Je nach Ausgestaltung der Halterung, auf die die Filtervorrichtung aufzusetzen ist, können entsprechend unterschiedliche Anordnungen der Stutzen vorgesehen werden.

Die Stutzen können auf einer gemeinsamen oder auf zueinander parallelen Längsebenen liegen.

Die vorliegende Erfindung betrifft ferner die Verwendung eines Gehäuses, an dem sämtliche Anschlüsse für in dem Gehäuse verfügbare Strömungsräume angeordnet sind, in einer Filtervorrichtung mit wenigstens zwei durch Membranen voneinander getrennten Strömungsräumen sowie mit wenigstens einem Endbereich, der mit einem der Strömungsräume in Verbindung steht und der durch eine auf das Gehäuse aufsetzbare Filterkappe verschließbar ist.

Weitere Einzelheiten und Vorteile der vorliegenden Erfindung werden anhand von in der Zeichnung dargestellten Ausführungsbeispielen näher erläutert. Es zeigen:
Fig. 1: eine schematische perspektivische Darstellung eines Gehäuses einer erfindungsgemäßen Filtervorrichtung,
Fig. 2: eine Seitenansicht einer erfindungsgemäßen Filtervorrichtung,
Fig. 3 eine Längsschnittdarstellung eines Gehäuses einer erfindungsgemäßen Filtervorrichtung,
Fig. 4: eine perspektivische Darstellung eines Gehäuses einer erfindungsgemäßen Filtervorrichtung mit nebeneinander angeordneten Anschlußstutzen,
Fig. 5: eine Querschnittsansicht des Gehäuses gemäß Fig. 4 sowie einer Halterung einer Dialysemaschine,
Fig. 6: eine perspektivische Darstellung eines Endbereiches eines Gehäuses einer erfindungsgemäßen Filtervorrichtung mit tangential verlaufenden Anschlußstutzen,
Fig. 7: eine schematische Längs- und Querschnittsansicht eines Endbereiches einer erfindungsgemäßen Filtervorrichtung mit tangentialen Anschlußstutzen und
Fig. 8: perspektivische Ansichten eines Gehäuses einer erfindungsgemäßen Filtervorrichtung mit tangentialen Anschlußstutzen.

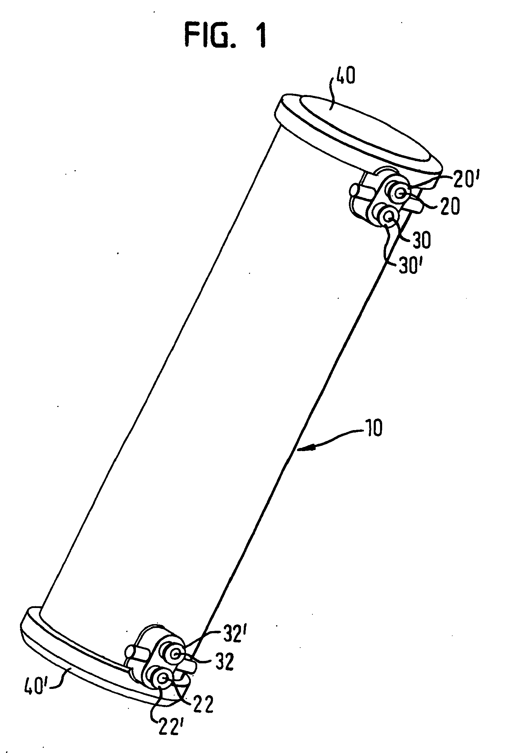

Fig. 1 zeigt in einer perspektivischen Darstellung das Gehäuse 10 einer erfindungsgemäßen Filtervorrichtung. Das Gehäuse 10 ist rohrabschnittförmig ausgeführt. Die beiden Endbereiche des Gehäuses sind mit Filterkappen 40, 40' verschlossen. Die Filterkappen 40, 40' weisen keinerlei Anschlüsse auf. Das Gehäuse 10 weist in beiden Endbereichen jeweils zwei Anschlüsse 20, 22 und 30, 32 auf, die als Anschlußstutzen 20', 22', 30', 32' ausgeführt sind. Jeweils ein Anschluß 20, 22 ist mit dem blutdurchströmten Raum der Filtervorrichtung und jeweils der andere Anschluß 30, 32 mit dem von Dialysat durchströmten Raum der Filtervorrichtung verbunden. Die Endbereiche der Stutzen 20', 22', 30', 32' liegen auf einer gemeinsamen Ebene und darüber hinaus auf einer gemeinsamen Längsebene. Die Position der Anschlüsse 20, 22, 30, 32 ist im Rahmen der Fertigung des Gehäuses 10 exakt festgelegt. Beim Anschluß der dargestellten Filtervorrichtung an eine entsprechende Halterung eines Dialysegerätes kann auf diese Weise eine genaue Positionierung und damit ein sehr gut dichtender Anschluß erreicht werden. Positionierungsprobleme, die bei vorbekannten Lösungen mit Anschlüsse aufweisenden Filterkappen auftreten, werden bei der erfindungsgemäßen Filtervorrichtung dadurch vermieden, daß die Filterkappen keine Anschlüsse aufweisen. Toleranzschwankungen bei der Herstellung oder Montage der Filterkappen 40, 40' auf das Gehäuse 10 spielen daher für die exakte Konnektion der Filtervorrichtung an einer Halterung eines Dialysegerätes keine Rolle.

In dem Gehäuse 10 ist ein nicht dargestelltes Hohlfaserbündel aufgenommen, das in den Endbereichen durch dichtend mit dem Gehäuse 10 in Verbindung stehende Vergußmassen fixiert ist. Die Vergußmassen trennen die durch die Filterkappen 40, 40' begrenzten, mit den Anschlüssen 20, 22 sowie mit den Durchgängen der Hohlfasern in Verbindung stehenden Blutkammern von dem von Dialysat durchströmten Faseraußenraum.

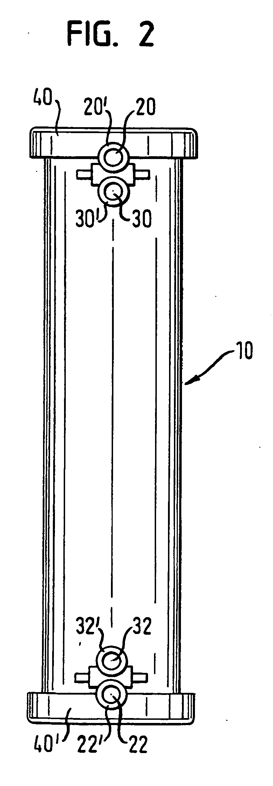

Fig. 2 zeigt in einer Seitenansicht eine Filtervorrichtung gemäß der vorliegenden Erfindung. Diese Filtervorrichtung entspricht der in Fig. 1 schematisch dargestellten.

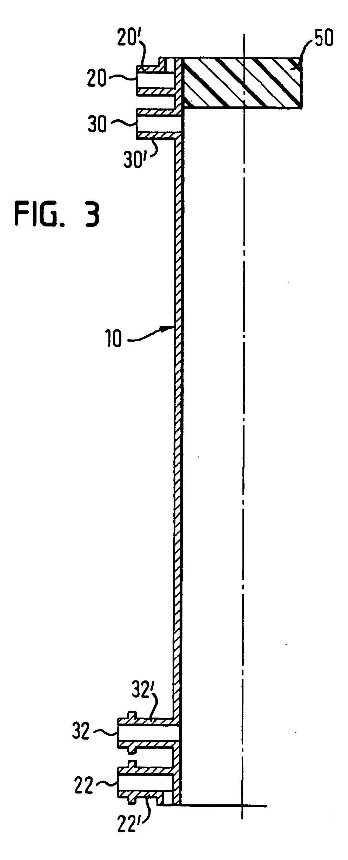

Fig. 3 zeigt in einer Längsschnittdarstellung die Anordnung der Stutzen 20', 22', 30', 32' am Gehäuse 10. Der in Fig. 3, rechts dargestellte schraffierte Bereich stellt die Vergußmasse 50 dar, die die Enden der Hohlfasern fixiert. Die beiden von den Endbereichen des Gehäuses 10 beabstandeten Stutzen 30', 32' dienen zur Zufuhr bzw. Abfuhr von Dialysat in bzw. aus dem das Hohlfaserbündel umgebenden Faseraußenraum. Die Stutzen 20', 22' dienen zur Zu- bzw. Abfuhr von Blut. Das Blut wird durch die Anschlüsse 20, 22 bzw. die Stutzen 20', 22' in bzw. aus der Filtervorrichtung ein- bzw. ausgeführt. Diese Anschlüsse stehen mit Blutkammern in Verbindung, die zwischen den Filterkappen und den Vergußmassen 50 gebildet werden und in die die offenen Enden der Fasern des Hohlfaserbündels münden.

Die Stutzen 20', 22' können mit dem Gehäuse 10 fest verbunden sein oder auch in eine entsprechende Aufnahme eingeschraubt sein.

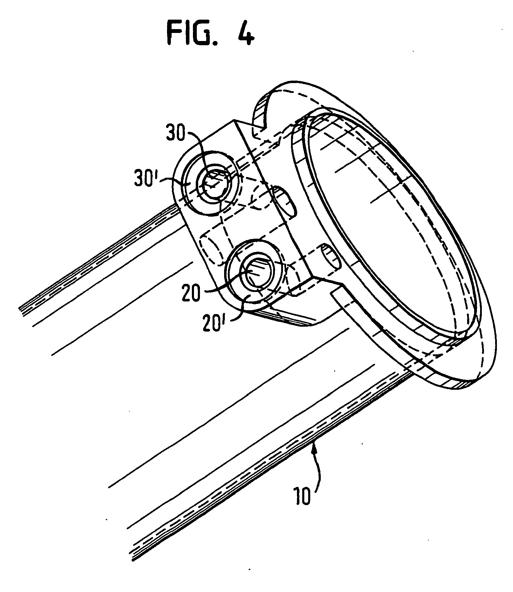

In Fig. 4 ist ein Endbereich eines Gehäuses 10 einer erfindungsgemäßen Filtervorrichtung dargestellt, bei der die Anschlüsse 20, 30 bzw. die Anschlußstutzen 20', 30' nebeneinander angeordnet sind. Die Anschlußstutzen 20', 30' werden hier durch einen am Gehäuse angeordneten Aufsatz gebildet, in dem sich Kanäle erstrecken, die die Anschlüsse 20, 30 mit dem Dialysatraum sowie mit der Blutkammer verbinden. Der Endbereich des Gehäuses 10 weist einen ringförmigen Flansch auf, auf den die nicht dargestellte Filterkappe aufgesetzt und dann mit dem Gehäuse verbunden wird.

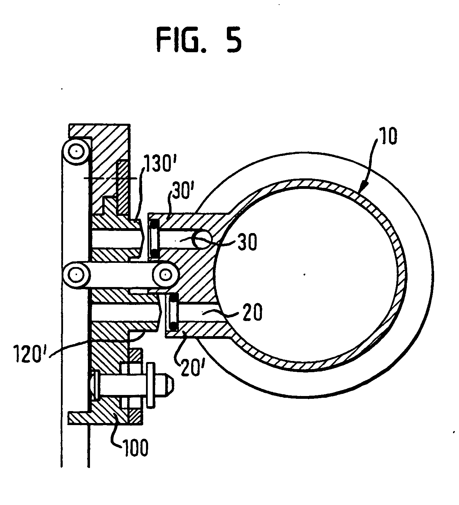

Fig. 5 zeigt das Gehäuse gemäß Fig. 4 sowie eine Halterung eines Dialysegerätes in einer Querschnittsansicht. Die Stutzen 20', 30' des Gehäuses 10 werden dichtend mit entsprechenden Gegenstücken 120', 130' der Halterung 100 verbunden. Aufgrund der fest vorgegebenen Anordnung der Anschlüsse bzw. Stutzen am Gehäuse 10 sind Korrektureinrichtungen an der Halterung, mittels derer die Position der entsprechenden Gegenstücke 120', 130' korrigiert werden müßte, nicht erforderlich.

Die dichtende Verbindung zwischen den Stutzen 20', 30' und den Gegenstücken 120', 130' wird in dem Ausführungsbeispiel gemäß Fig. 5 durch in den Stutzen 20', 30' aufgenommene O-Ring-Dichtungen bewirkt.

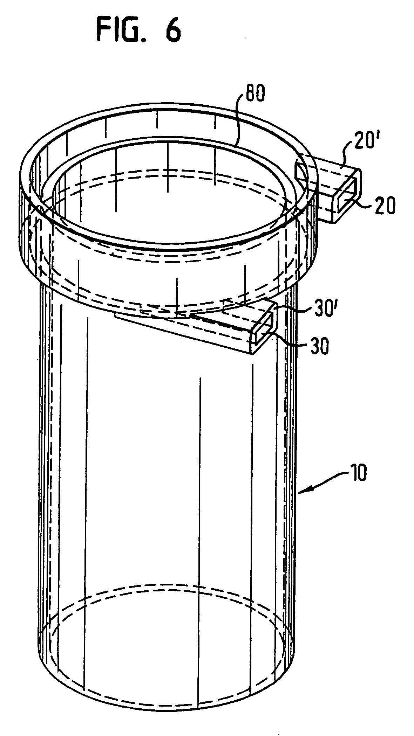

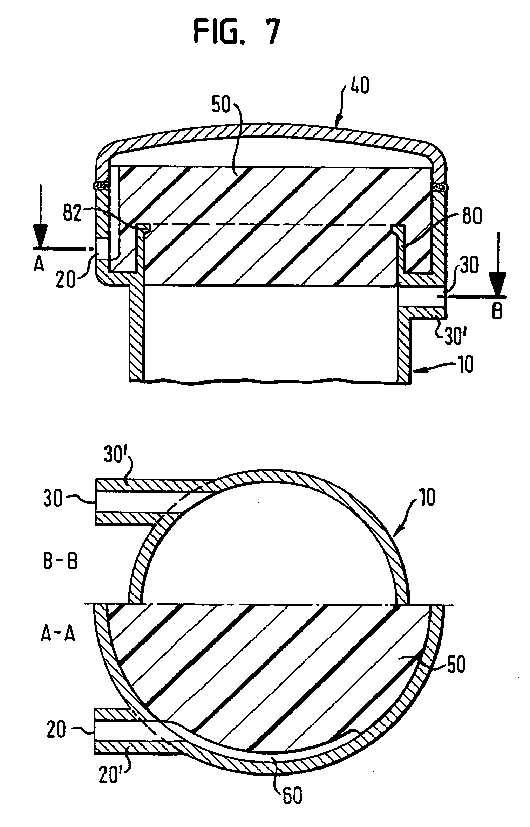

Fig. 6 zeigt in einer perspektivischen Darstellung ein Gehäuse 10 mit tangential ausgeführten Stutzen 20', 30', die Anschlüsse 20, 30 aufweisen, von denen jeweils einer mit der blutdurchströmten Seite und einer mit der dialysatdurchströmten Seite der Filtervorrichtung in Verbindung steht. Der Endbereich des Gehäuses 10 ist in seinem Durchmesser vergrößert und weist einen frei auskragenden, ringförmigen Vorsprung 80 kleineren Durchmessers auf, der in eine ein Hohlfaserbündel aufnehmende, in Fig. 6 nicht dargestellte Vergußmasse eingebettet ist. Dies ist im Detail in Fig. 7 dargestellt, wobei Fig. 7, oben eine Schnittdarstellung in Längsrichtung und Fig. 7, unten eine Schnittdarstellung in Querrichtung zeigt.

Fig. 7 zeigt die Filtervorrichtung in einer schematischen und vereinfachten Längs- und Querschnittsdarstellung. In der Längsschnittdarstellung gemäß Fig. 7, oben sind die Außenkonturen der Filtervorrichtung nicht dargestellt. Ferner ist der Stutzen 20' in Fig. 7, oben nicht gezeigt.

Wie aus Fig. 7, oben hervorgeht, ist der ringförmige Vorsprung 80 des Gehäuses 10 in der Vergußmasse 50 eingebettet. Die Dichtung zwischen der Blut- und Dialysatseite erfolgt durch Anliegen der Vergußmasse 50 an dem zylinderförmigen Bereich 80. Dieser weist einen Hinterschnittkragen 82 auf, der sich in der Vergußmasse 50 erstreckt. Aufgrund der durch das Schrumpfen der Vergußmasse 50 bedingten Vorspannung liegt die Vergußmasse 50 dichtend an dem Vorsprung 80 an. Die Dichtung zwischen Blut- und Dialysatseite erfolgt hier nur durch Eingießen des Vorsprungs 80 mit zwangsentformten Hinterschnittkragen 82 in die Vergußmasse 50. Der Vorsprung 80 kann durch Plasmabehandlung haftfähiger gemacht werden. Er kann nicht nur die dargestellte Ringform, sondern auch andere Geometrien, wie z.B. einen "Faltenstern" umfassen. Die Dichtung erfolgt anders als bei vorbekannten Lösungen nicht durch Haftung, sondern durch Anliegen des Vergusses 50 an dem Vorsprung 80 mit der durch den Vergußschrumpf bedingten Vorspannung.

Die Anschlußstutzen 20', 30' für Blut und Dialysat sind tangential am Gehäuse 10 angeordnet, wie sich dies aus Fig. 7, unten ergibt. Die Querschnittsdarstellung gemäß Linie B-B ist in Fig. 7, unten gegenüber der Darstellung in Fig. 7, oben um 180° gedreht. Die Schnittlinie des Vorsprungs 80 ist in Fig. 7, unten (Schnitt A-A) nicht dargestellt. In der Vergußmasse 50 ist ein Blutführungskanal 60 angeordnet, der sich an den Blutanschlußstutzen 20' anschließt und der eine Verbindung des Blutanschlußstutzens 20' mit der Oberseite der Vergußmasse 50 und damit mit den offenen Endbereichen der Hohlfasern herstellt. Darüber hinaus kann der Blutführungskanal 60 dazu dienen, das zugeführte Blut möglichst gleichmäßig über die Oberfläche der Vergußmasse 50 zu verteilen. Hierzu kann der Blutführungskanal 60 spiralförmig ansteigend ausgebildet sein.

Das Gehäuse 10 ist durch die Filterkappe 40 verschlossen, die stumpf durch eine Schweißung mit dem Gehäuse 10 verbunden ist. Durch die Filterkappe 40 wird eine Blutkammer oberhalb der Vergußmasse 50 gebildet, aus der das Blut in bzw. aus den Hohlräumen der Hohlfasern geführt wird.

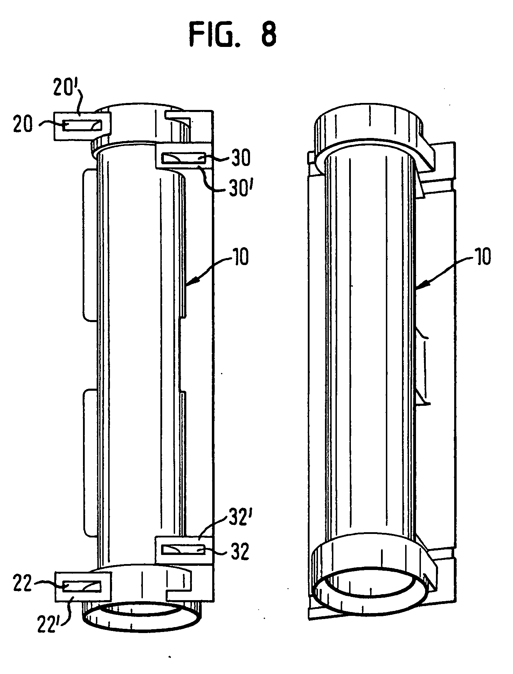

Wie aus Fig. 7 weiter hervorgeht, weist die Filterkappe 40 keinerlei Anschlüsse auf. Fig. 8 zeigt schließlich perspektivische Darstellungen des Gehäuses 10 der erfindungsgemäßen Filtervorrichtung. Hieraus wird nochmals deutlich, daß sämtliche Anschlüsse 20, 22, 30, 32 bzw. die entsprechenden Anschlußstutzen 20', 22', 30', 32' am Gehäuse 10 angeordnet sind. Die aufschweißbaren Filterkappen dienen - wie oben ausgeführt - zur Abgrenzung der Blutkammern. Sie können darüber hinaus weitere Funktionselemente wie z. B. Luftabscheidemembranen aufweisen.

Die erfindungsgemäße Filtervorrichtung ermöglicht nicht nur eine einfache Konnektion mit einer entsprechende Gegenstücke aufweisende Halterung eines Dialysegerätes, sondern ermöglicht es darüber hinaus, ein hochintegriertes Disposable zur Verfügung zu stellen. Dieses kann Funktionselemente, wie Pumpen, Filter, Meßvorrichtungen, Steuerungseinrichtungen, Vorrichtungen zum Temperieren, zur Entlüftung etc. aufweisen. Aufgrund der Anordnung dieser Funktionselemente in oder an dem Gehäuse entfallen Dichtungsprobleme, die bei vorbekannten Lösungen bei der Konnektion dieser Funktionselemente außerhalb des Gehäuses aufgetreten sind. Bei vorbekannten Lösungen sind derartige Funktionselemente in ein komplexes Schlauchsystem integriert, was aufgrund der zahlreichen Konnektionen zu Dichtungsproblemen führen kann und darüber hinaus in der Herstellung sowie im Betrieb aufwendig ist. Bei der erfindungsgemäßen Lösung kann dieses Problem durch die Integration der Funktionselemente in bzw. an die Filtervorrichtung gelöst werden. Hierzu werden keine Konnektoren mehr benötigt, so daß entsprechende Produktionsschwierigkeiten (Assemblierung, Sterilisationsfestigkeit) entfallen.

Als weiterer Vorteil ergibt sich, daß die vollautomatische Produktion der erfindungsgemäßen Filtervorrichtung wesentlich einfacher wird, da die Toleranzen bei der Herstellung bzw. beim Aufsetzen der Filterkappen für die Genauigkeit der Konnektierung keine Rolle spielen. Aus dem gleichen Grund wird eine automatische Konnektion des Disposables an die Halterung der Dialysemaschine ermöglicht, was in einer entsprechend einfacheren Handhabung resultiert.

## Patentansprüche

1. Filtervorrichtung mit einem Gehäuse (10), mit wenigstens zwei durch Membranen voneinander getrennten Strömungsräumen, mit mindestens einem Anschluß (20, 22, 30, 32) für jeden Strömungsraum, sowie mit wenigstens einer Filterkappe (40, 40'), die im Endbereich des Gehäuses (10) aufgesetzt ist und mit diesem mittelbar oder unmittelbar verbunden ist,
**dadurch gekennzeichnet,**
**daß** sämtliche Anschlüsse (20, 22, 30, 32) für die Strömungsräume an dem Gehäuse (10) angeordnet sind.

2. Filtervorrichtung nach Anspruch 1, **dadurch gekennzeichnet, daß** das Gehäuse (10) rohrabschnittförmig ausgeführt ist und daß zwei Filterkappen (40, 40') vorgesehen sind, die auf beide Endbereiche des Gehäuses (10) aufgesetzt und mit diesem verbunden sind.

3. Filtervorrichtung nach Anspruch 1 oder 2, **dadurch gekennzeichnet, daß** je Strömungsraum zwei Anschlüsse (20, 22, 30, 32) vorgesehen sind.

4. Filtervorrichtung nach Anspruch 3, **dadurch gekennzeichnet, daß** in den Endbereichen des Gehäuses (10) je zwei Anschlüsse (20, 22, 30, 32) angeordnet sind, die in Längsrichtung des Gehäuses (10) jeweils übereinander oder nebeneinander angeordnet sind.

5. Filtervorrichtung nach einem der Ansprüche 1 bis 4, **dadurch gekennzeichnet, daß** die Anschlüsse (20, 22, 30, 32) Stutzen (20', 22', 30', 32') aufweisen, die sich relativ zum Gehäuse (10) in radialer oder tangentialer Richtung erstrecken.

6. Filtervorrichtung nach Anspruch 5, **dadurch gekennzeichnet, daß** die Anschlußstutzen (20', 22') tangential ausgeführt sind und in einen Führungskanal (60) münden, der sich zumindest teilumfangsförmig um eine das Hohlfaserbündel der Filtervorrichtung einfassende Vergußmasse (50) erstreckt.

7. Filtervorrichtung nach einem der Ansprüche 1 bis 6, **dadurch gekennzeichnet, daß** die Endbereiche des Gehäuses (10) im Durchmesser vergrößert sind und einen frei auskragenden Vorsprung (80) kleineren Durchmessers aufweisen, der in eine ein Hohlfaserbündel einfassende Vergußmasse (50) eingebettet ist.

8. Filtervorrichtung nach einem der Ansprüche 1 bis 7, **dadurch gekennzeichnet, daß** die Filterkappen (40, 40') mit dem Gehäuse (10) verklebt, verschweißt oder verschraubt sind.

9. Filtervorrichtung nach einem der Ansprüche 1 bis 8, **dadurch gekennzeichnet, daß** in oder an dem Gehäuse (10) Funktionselemente integriert sind, mittels derer Eigenschaften der durch die Filtervorrichtung geführten Medien erfaßbar und/oder veränderbar sind oder der Fluß durch die Filtervorrichtung beeinflußbar ist.

10. Filtervorrichtung nach Anspruch 9, **dadurch gekennzeichnet, daß** die Funktionselemente Kanäle, Ventile, Pumpen, Meßkammern, Filter und Entlüftungskammern und -membranen umfassen.

11. Filtervorrichtung nach Anspruch 1 bis 10, **dadurch gekennzeichnet, daß** das Gehäuse (10) sowie die Filterkappen (40, 40') aus Polypropylen gefertigt sind.

12. Filtervorrichtung nach einem der Ansprüche 1 bis 11, **dadurch gekennzeichnet, daß** die Anschlüsse (20, 22, 30, 32) Stutzen (20', 22', 30', 32') aufweisen, deren Endbereiche in zueinander parallelen Ebenen oder in einer gemeinsamen Ebene liegen.

13. Filtervorrichtung nach einem der Ansprüche 1 bis 12, **dadurch gekennzeichnet, daß** die Anschlüsse (20, 22, 30, 32) Stutzen (20', 22', 30', 32') aufweisen, die auf einer gemeinsamen oder auf zueinander parallelen Längsebenen liegen.

14. Verwendung eines Gehäuses (10) bei dem jeder der Strömungsräume über mindestens einen Anschluß (20, 22, 30, 32) verfügt und sämtliche Anschlüsse (20, 22, 30, 32) für die Strömungsräume an dem Gehäuse (10) angeordnet sind, in einer Filtervorrichtung nach einem der Ansprüche 1 bis 13.

15. Verwendung nach Ansprüche 14, **dadurch gekennzeichnet, daß** das Gehäuse (10) nach einem der Ansprüche 3 bis 7 oder 9 bis 13 ausgeführt ist.

## Claims

1. A filter device comprising a housing (10), at least two flow chambers separated from each other by membranes, at least one connection (20, 22, 30, 32) for each flow chamber, and at least one filter cap (40, 40') which is fitted in the end region of the housing (10) and is indirectly or directly connected thereto,
**characterised in that**
all connections (20, 22, 30, 32) for the flow chambers are arranged on the housing (10).

2. A filter device according to claim 1 **characterised in that** the housing (10) is in the form of a tubular portion and there are provided two filter caps (40, 40') which are fitted onto both end regions of the housing (10) and are connected to the housing.

3. A filter device according to claim 1 or claim 2 **characterised in that** there are provided two connections (20, 22, 30, 32) for each flow chamber.

4. A filter device according to claim 3 **characterised in that** arranged in each of the end regions of the housing (10) are two respective connections (20, 22, 30, 32) which are respectively arranged in mutually superposed or juxtaposed relationship in the longitudinal direction of the housing (10).

5. A filter device according to one of claims 1 to 4 **characterised in that** the connections (20, 22, 30, 32) have connecting portions (20', 22', 30', 32') which extend in the radial or tangential direction relative to the housing (10).

6. A filter device according to claim 5 **characterised in that** the connecting portions (20', 22') are provided tangentially and open into a guide passage (60) which extends at least in partially peripheral relationship around a casting material (50) enclosing the hollow fibre bundle of the filter device.

7. A filter device according to one of claims 1 to 6 **characterised in that** the end regions of the housing (10) are enlarged in diameter and have a freely protruding projection (80) of smaller diameter, which is embedded in a casting material (50) enclosing a hollow fibre bundle.

8. A filter device according to one of claims 1 to 7 **characterised in that** the filter caps (40, 40') are glued, welded or screwed to the housing (10).

9. A filter device according to one of claims 1 to 8 **characterised in that** integrated in or on the housing (10) are functional elements, by means of which properties of the media guided through the filter device can be detected and/or altered or the flow through the filter device can be influenced.

10. A filter device according to claim 9 **characterised in that** the functional elements include passages, valves, pumps, measuring chambers, filters and venting chambers and diaphragms.

11. A filter device according to claims 1 to 10 **characterised in that** the housing (10) and the filter caps (40, 40') are made from polypropylene.

12. A filter device according to one of claims 1 to 11 **characterised in that** the connections (20, 22, 30, 32) have connecting portions (20', 22', 30', 32'), the end regions of which are disposed in mutually parallel planes or in a common plane.

13. A filter device according to one of claims 1 to 12 **characterised in that** the connections (20, 22, 30, 32) have connecting portions (20', 22', 30', 32') which are disposed on a common longitudinal plane or on mutually parallel longitudinal planes.

14. Use of a housing (10) in which each of the flow chambers has at least one connection (20, 22, 30, 32) and all connections (20, 22, 30, 32) for the flow chambers are arranged on the housing (10), in a filter device according to one of claims 1 to 13.

15. Use according to claim 14 **characterised in that** the housing (10) is designed in accordance with one of claims 3 to 7 or 9 to 13.

## Revendications

1. Dispositif de filtration avec un boîtier (10), avec au moins deux enceintes d'écoulement séparées l'une de l'autre par des membranes, avec au moins un raccord (20, 22, 30, 32) pour chaque enceinte d'écoulement, et avec au moins un capuchon de filtre (40, 40') qui est mis en place dans la zone d'extrémité du boîtier (10) et est relié à celui-ci directement ou indirectement,
**caractérisé**
**en ce que** tous les raccords (20, 22, 30, 32) pour les enceintes d'écoulement sont disposés au boîtier (10).

2. Dispositif de filtration selon la revendication 1, **caractérisé en ce que** le boîtier (10) est réalisé en une forme de section tubulaire, et **en ce que** deux capuchons de filtre (40, 40') sont prévus qui sont placés sur les deux zones d'extrémité du boîtier (10) et sont reliés à celui-ci.

3. Dispositif de filtration selon la revendication 1 ou 2, **caractérisé en ce que** deux raccords (20, 22, 30, 32) sont prévus par enceinte d'écoulement.

4. Dispositif de filtration selon la revendication 3, **caractérisé en ce que** sont disposés dans les zones d'extrémité du boîtier (10) respectivement deux raccords (20, 22, 30, 32) qui sont disposés dans la direction longitudinale du boîtier (10) respectivement les uns au-dessus des autres ou les uns à côté des autres.

5. Dispositif de filtration selon l'une des revendications 1 à 4, **caractérisé en ce que** les raccords (20, 22, 30, 32) présentent des manchons (20', 22', 30', 32') qui s'étendent relativement au boîtier (10) dans la direction radiale ou tangentielle.

6. Dispositif de filtration selon la revendication 5, **caractérisé en ce que** les manchons de raccordement (20', 22') sont réalisés tangentiellement et débouchent dans un canal de guidage (60) qui s'étend au moins selon une forme périphérique partielle autour d'une masse de scellement (50) renfermant le faisceau de fibres creuses du dispositif de filtration.

7. Dispositif de filtration selon l'une des revendications 1 à 6, **caractérisé en ce que** les zones d'extrémité du boîtier (10) ont été agrandies en diamètre et présentent une saillie dépassant librement (80) d'un plus petit diamètre qui est noyée dans une masse de scellement (50) renfermant un faisceau de fibres creuses.

8. Dispositif de filtration selon l'une des revendications 1 à 7, **caractérisé en ce que** les capuchons de filtre (40, 40') sont assemblés avec le boîtier (10) par collage, soudage ou vissage.

9. Dispositif de filtration selon l'une des revendications 1 à 8, **caractérisé en ce que** des éléments fonctionnels sont intégrés dans ou au boîtier (10) au moyen desquels des caractéristiques des milieux guidés à travers le dispositif de filtration peuvent être détectées et/ou modifiées ou bien le flux à travers le dispositif de filtration peut être influencé.

10. Dispositif de filtration selon la revendication 9, **caractérisé en ce que** les éléments fonctionnels comprennent des canaux, vannes, pompes, enceintes de mesure, filtres et enceintes et membranes de désaération.

11. Dispositif de filtration selon la revendication 1 à 10, **caractérisé en ce que** le boîtier (10) ainsi que les capuchons de filtre (40, 40') sont fabriqués en polypropylène.

12. Dispositif de filtration selon l'une des revendications 1 à 11, **caractérisé en ce que** les raccords (20, 22, 30, 32) présentent des manchons (20', 22', 30', 32') dont les zones d'extrémité se situent dans des plans parallèles les uns aux autres ou dans un plan commun.

13. Dispositif de filtration selon l'une des revendications 1 à 12, **caractérisé en ce que** les raccords (20, 22, 30, 32) présentent des manchons (20', 22', 30', 32') qui se situent sur un plan longitudinal commun ou sur des plans longitudinaux parallèles les uns aux autres.

14. Utilisation d'un boîtier (10), dans lequel chacune des enceintes d'écoulement dispose d'au moins un raccord (20, 22, 30, 32) et tous les raccords (20, 22, 30, 32) pour les enceintes d'écoulement sont disposés au boîtier (10), dans un dispositif de filtration selon l'une des revendications 1 à 13.

15. Utilisation selon la revendication 14, **caractérisé en ce que** le boîtier (10) est réalisé selon l'une des revendications 3 à 7 ou 9 à 13.
